# EUROPÄISCHE PATENTANMELDUNG

(11) **EP 1 859 728 A2**
(43) Veröffentlichungstag der Anmeldung: **28.11.2007**
(21) Anmeldenummer: 07010219.9
(22) Anmeldetag: 23.05.2007
(51) Int. Cl.: A61B 1/267

(54) **Endoskop insbesondere zur Intubation eines Atemwegs**

(30) Priorität: 24.05.2006 DE 102006025621
(71) Anmelder: Karl Storz GmbH & Co. KG, 78532 Tuttlingen (DE)
(72) Erfinder: Renner, Martin, 78576 Liptingen (DE)
(74) Vertreter: Heuckeroth, Volker

(57) **Zusammenfassung**

Ein. Endoskop (10) insbesondere zur Intubation eines Atemwegs weist ein Schaft (14) und ein Okular (30) am proximalen Ende eines Endoskopkopfes (12) und ein Gelenk (32) zwischen dem Schaft (14) und dem Okular (30) zum Abwinkeln des Okulars (30) relativ zu einer Längsrichtung (34) des Schafts (14) auf. Das Gelenk (32) ist so ausgebildet, dass das Okular (30) in einer ersten Ebene abwinkelbar ist. Darüber hinaus ist das Gelenk (32) so ausgebildet, dass das Okular (30) zumindest in einer zweiten Ebene, die quer zur ersten Ebene verläuft, abwinkelbar ist (siehe Fig. 1).

## Beschreibung

Die Erfindung betrifft ein Endoskop, insbesondere zur Intubation eines Atemwegs, mit einem Schaft und einem Okular am proximalen Ende eines Endoskopkopfes, und mit einem Gelenk zwischen dem Schaft und dem Okular zum Abwinkeln des Okulars relativ zu einer Längsrichtung des Schafts, wobei das Gelenk so ausgebildet ist, dass das Okular in einer ersten Ebene abwinkelbar ist.

Ein derartiges Instrument ist beispielsweise aus dem DE-Firmenprospekt der Karl Storz GmbH & Co. KG, Tuttlingen, "Karl Storz-Endoskope, Endoskope für Anästhesie und Notfallmedizin", 3. Ausgabe 1/2004, Seite AN-SET 9 B, bekannt. Aus diesem Prospekt ist ein mit der Instrumentennummer 10330 B versehenes Endoskop bekannt.

Das bekannte Endoskop wird zur Intubation der Atemwege eines Menschen verwendet, wobei das distale Ende des Endoskops durch die Nase oder den Mund eines Patienten in die Luftröhre zur optischen Kontrolle der Einführung eines Intubationsschlauchs eingeführt wird. Das Endoskop weist einen Endoskopschaft und einen Endoskopkopf auf, wobei ein Okular am proximalen Ende des Endoskopkopfs angeordnet ist. Das Okular und der Schaft sind über ein Gelenk miteinander verbunden.

Ein Gelenk im Sinne der vorliegenden Erfindung weist zumindest zwei Elemente auf, die durch Rotation und/oder Translation aufeinander wirken.

Der Endoskopkopf des bekannten Endoskops weist ein zweiteiliges Gehäuse auf, wobei das Gelenk zwischen beiden Gehäuseteilen angeordnet ist und das proximale Ende des Schafts in gleicher Längsrichtung wie das distale Ende des Endoskopkopfs verläuft. Die Längsrichtung des Endoskopschafts bezeichnet im Folgenden diejenige Richtung, die durch die Längsrichtung des proximalen Schaftendes definiert ist. Das Okular ist aus einer Nullgradstellung heraus abwinkelbar, die beispielsweise mit der Längsrichtung des Schafts übereinstimmen kann.

Beleuchtungslicht wird am distalen Ende des Endoskopkopfes distalseitig des Gelenks über einen Anschluss in ein Lichtleitsystem eingekoppelt, das sich bis zum distalen Ende des Schafts erstreckt. Beobachtungslicht des bildgebenden Systems wird vom distalen Ende des Schafts über ein Bildleitsystem durch das Gelenk hindurch zum Okular geleitet. Das Bildleitsystem weist geordnete Lichtleitfasern und/oder ein Linsensystem zur Lichtleitung und Fokussierung auf. Das Bildleitsystem kann als semi-flexibler oder flexibler Lichtleiter ausgebildet sein.

Solche Endoskope kommen bevorzugt in der Notfallmedizin zum Einsatz. Ein Arzt, der sich in gebeugter Körperhaltung über einem Patienten befindet, hält das Endoskop an seinem Endoskopkopf, wobei das distale Ende des Schafts in die Luftröhre des Patienten eingeführt wird. Hierbei befindet sich eine Hand des Arztes am proximalen Ende des Endoskopkopfes und die andere Hand am distalen Ende des Endoskopkopfes, um das Okular relativ zur Längsrichtung des Schafts abwinkeln zu können. Das abwinkelbar ausgebildete Okular ermöglicht es dem Arzt, eine solche Position zum Patienten einzunehmen, dass er sich während des Eingriffs nicht direkt über den Nasen- und Mundöffnungen des Patienten befinden muss.

Allerdings ist das Okular des aus dem Stand der Technik bekannten Endoskops nur in einer definierten Ebene relativ zur Längsrichtung des Schafts abwinkelbar, so dass die Bewegungsfreiheit des Arztes eingeschränkt ist. In einer Situation, in der die Stellung des Arztes zum Patienten derart ist, dass sich der Arzt zumindest teilweise über dem Patient befindet, muss der Arzt in körperlich anstrengender und für ihn umständlicher Haltung während des gesamten Eingriffs verbleiben. Falls für den Arzt keine akzeptable Ausrichtung des Okulars zum Schaft gefunden werden kann, muss das Endoskop entweder erneut in die Luftröhre eingeführt werden oder der Patient in eine günstige Position gedreht werden. Hierbei besteht die Gefahr, dass dem Patienten zusätzlich Verletzungen zugefügt werden. Eine Drehung des Endoskops um die Längsrichtung des Schafts ist bei einem semi-flexiblen Schaft oder gar starren Schaft mit gekrümmten Verlauf nicht möglich, wenn das Endoskop in die Luftröhre eingeführt ist.

Ferner ist aus DE 103 51 185 A1 ein Endoskop mit einem biegeflexiblen Biegeabschnitt bekannt, wobei das Endoskop zwischen dem distalen Schaftende und dem proximalen Ende des Endoskopkopfes einen Arbeitskanal zur Durchführung eines Lichtleitsystems und Instrumentenkanäle aufweist. Das Okular, das am proximalen Ende des Endoskopkopfes sitzt, kann über den Biegeabschnitt aus seiner Geradstellung abgebogen werden. Der Biegeabschnitt ist als ein einteiliges Rohr mit einer Spiralverstärkung entlang seiner Längserstreckung zwischen einem festen Endstück am proximalen Ende des Endoskopkopfes und einem Gehäuse des Endoskopkopfes ausgebildet. Ein Biegeabschnitt hat gegenüber einem Gelenk den Nachteil, dass er nach vielen Biegewechseln ermüden kann, d.h. nach längerem Gebrauch des Endoskops erschlaffen kann. Nach mehreren Richtungswechseln der Abwinkelung des Okulars kann der Biegeabschnitt ungleichmäßige, schlangenförmig verbogene Bereiche aufweisen, weil keine feste Biegestelle definiert ist. Derartige verbogene Bereiche des Biegeabschnitts, durch den das Bildleitsystem für die Beobachtung hindurchgeht, führen zu einem entsprechenden schlangenförmigen Verlauf des Lichtleitsystems, die die Abbildungsqualität des Bildleitsystems beeinträchtigen können.

Der Erfindung liegt die Aufgabe zugrunde, ein Endoskop der eingangs genannten Art zu schaffen, das dem Arzt eine größere Bewegungsfreiheit bei der Beobachtung durch das Endoskop erlaubt.

Erfindungsgemäß wird diese Aufgabe hinsichtlich des eingangs genannten Endoskops dadurch gelöst, dass das Gelenk, das zwischen dem Schaft und dem Okular angeordnet ist, so ausgebildet ist, dass das Okular zumindest in einer zweiten Ebene, die quer zur ersten Ebene verläuft, abwinkelbar ist.

Demnach erlaubt das erfindungsgemäße Endoskop nicht nur die Abwinklung des Okulars in einer definierten Ebene relativ zur Längsrichtung des Schafts. Vielmehr ist das Okular räumlich relativ zur Längsrichtung des Schafts abwinkelbar ausgebildet. Das erfindungsgemäße Endoskop bietet vorteilhafterweise dem Arzt eine erweiterte Bewegungsfreiheit während des Eingriffs, da die relative Ausrichtbarkeit des Okulars zum Schaft um zumindest einen zweiten Winkelbereich, der durch eine zweite Ebene quer zur ersten Ebene gegeben ist, vergrößert ist. Zudem kann der Arzt ermüdungsfrei arbeiten, da er in einer körperlich entspannten Haltung während des Eingriffs verbleiben kann.

Ein weiterer Vorteil des erfindungsgemäßen Endoskops liegt in seiner einfachen Handhabbarkeit während des Eingriffs, so dass auch Ärzte ohne langjährige Berufserfahrung im Umgang mit Endoskopen das Endoskop der vorliegenden Erfindung verwenden können. Aufgrund der räumlichen Abwinkelbarkeit des Endoskops muss während der Einführung des Endoskops nicht darauf geachtet werden, wie das abgewinkelte Okular zur Längsrichtung des Schafts angeordnet ist, um dem Arzt eine günstige Stellung zum Patienten zu ermöglichen. Der Arzt kann noch nach Einführen des Endoskops die für ihn geeignetste Stellung zum Patienten einnehmen, indem er das Okular in die gewünschte Richtung abwinkelt.

Darüber hinaus ermöglicht das vorliegende Endoskop, das insbesondere im Bereich der Notfallmedizin eingesetzt werden kann, eine zeitsparende Anwendung während des Eingriffs. Ist das Endoskop bereits in die Luftröhre des Patienten eingeführt und die Stellung des Patienten zum Arzt für die weitere Behandlung nicht geeignet, kann, ohne das Endoskop erneut in die Luftröhre einzuführen, das Okular relativ zum Schaft in die günstigste räumliche Lage abgewinkelt werden.

In einer bevorzugten Ausgestaltung der Erfindung ist das Gelenk des Endoskops so ausgebildet, dass das Okular zumindest in einer der ersten oder zweiten Ebene bis zu einem Winkel von etwa 50°, vorzugsweise bis zu einem Winkel von etwa 40° und weiter vorzugsweise bis zu einem Winkel von etwa 30° relativ zur Längsrichtung des Schafts abwinkelbar ist.

Diese Maßnahme hat den Vorteil, dass der maximale Winkelbereich einer der beiden Ebenen, der durch die Abwinkelung des Okulars zu beiden Seiten relativ zur Längsrichtung des Schafts gegeben ist, 100°, 80° beziehungsweise 60° beträgt und dem Arzt eine große Bewegungsfreiheit einräumt.

In einer weiteren bevorzugten Ausgestaltung ist das Gelenk als Kugelgelenk ausgebildet.

Diese Maßnahme hat den Vorteil, dass das Gelenk konstruktiv einfach und kostengünstig hergestellt werden kann. Zudem erlaubt das Kugelgelenk eine bedienungsfreundliche Handbarkeit des Endoskops, da die gewünschte Abwinkelstellung des Okulars relativ zur Längsrichtung des Schafts auf direktem Weg erreichbar ist. Der Arzt muss während des Intubierens nicht überlegen, in welchen Winkelschritten das Okular abgewinkelt werden muss.

In einer weiteren bevorzugten Ausgestaltung weist das Kugelgelenk ein zweiteiliges Gehäuse auf, wobei ein erstes Gehäuseteil mit dem Schaft und ein zweites Gehäuseteil mit dem Okular verbunden ist.

Diese Maßnahme hat den Vorteil, dass das Gehäuse einen stabilen kompakten Schutz für das Lichtleitsystem bietet. Zudem ist der Endoskopkopf für den Arzt handlich und gut greifbar auf konstruktiv einfache Weise ausgebildet. Durch die Zweiteilung des Gehäuses wird gleichzeitig die Funktion der räumlichen Abwinkelung relativ zur Längsrichtung des Schafts realisiert.

In einer weiteren bevorzugten Ausgestaltung ist das erste Gehäuseteil mit Reibschluss gleitend in dem zweiten Gehäuseteil aufgenommen.

Diese Maßnahme hat den Vorteil, dass beide Gehäuseteile ohne weiteren technischen Aufwand zueinander bewegbar sind. Durch den Reibschluss wird eine Selbsthemmung des Gelenks geschaffen, so dass das Okular seine eingestellte räumliche Abwinkelung beibehält, auch wenn der Arzt das Okular loslässt.

In einer weiteren bevorzugten Ausgestaltung weist das erste Gehäuseteil eine kugelförmige Außenfläche und das zweite Gehäuseteil eine kugelförmige Innenfläche auf, in der das erste Gehäuseteil aufgenommen ist.

Diese Maßnahme hat den Vorteil, dass die Oberfläche beider Gehäuseteile glatt und ohne Vorsprünge ausgebildet ist und so die Bewegung beider Gehäuseteile zueinander nicht eingeschränkt wird. Da sowohl die Außenfläche des ersten Gehäuseteils als auch die Innenfläche des zweiten Gehäuseteils kugelförmig ausgebildet sind, sind beide Gehäuseteile passgenau miteinander verbunden. Zudem erlaubt die kugelförmige Ausgestaltung beider Gehäuseteile die Abwinkelung des Okulars relativ zur Längsrichtung des Schafts in mehr als einer zweiten Ebene quer zur ersten Ebene. Hierdurch kann das Okular auf einer Kugelschale bewegt werden.

In einer weiteren bevorzugten Ausgestaltung ist das erste Gehäuseteil derart in dem zweiten Gehäuseteil angeordnet, dass sich das zweite Gehäuseteil über den maximalen Außendurchmesser der kugelförmigen Außenfläche des ersten Gehäuseteils hinweg erstreckt.

Diese Maßnahme hat den Vorteil, dass beide Gehäuseteile ohne weitere technische Maßnahmen aneinander befestigt sind.

In einer weiteren bevorzugten Ausgestaltung weist die kugelförmige Außenfläche des ersten Gehäuseteils eine radiale Einschnürung auf einer dem zweiten Gehäuseteil abgewandten Seite auf.

Diese Maßnahme hat den Vorteil, dass die radiale Einschnürung den Bewegungsbereich des zweiten Gehäuseteils bezüglich des ersten Gehäuseteils erweitert, da das zweite Gehäuseteil weiter über die Außenfläche des ersten Gehäuseteils bewegt werden kann und nicht an ihr anstößt.

In einer weiteren bevorzugten Ausgestaltung verjüngt sich die radiale Einschnürung in Längsrichtung vom Gelenk weg gesehen.

Diese Maßnahme hat den Vorteil, dass die in Längsrichtung des Schafts gesehen radial kleiner werdende Einschnürung den maximalen Bewegungsbereich des zweiten Gehäuseteils bezüglich des ersten Gehäuseteils vergrößert.

In einer weiteren bevorzugten Ausgestaltung wirken beide Gehäuseteile als Anschlag zur Begrenzung der Abwinkelung des Okulars zusammen.

Diese Maßnahme hat den Vorteil, dass der maximale Ablenkwinkel des Okulars relativ zur Längsrichtung des Schafts begrenzt ist und das durch das Gelenk geführte Bildleitsystem nicht durch eine übermäßige Abwinkelung durchgebrochen werden kann.

In einer zum Kugelgelenk alternativen bevorzugten Ausgestaltung ist das Gelenk als Kardangelenk ausgebildet.

Auch in Form eines Kardangelenks kann das Gelenk technisch einfach und kostengünstig hergestellt werden, um eine räumliche Abwinkelung des Okulars zu schaffen.

In einer weiteren bevorzugten Ausgestaltung weist das Kardangelenk ein zweiteiliges Gehäuse und ein Achsenkreuz auf, wobei ein erstes Gehäuseteil mit dem Schaft und ein zweites Gehäuseteil mit dem Okular verbunden ist und das Achsenkreuz zwischen den beiden Gehäuseteilen angeordnet ist.

Diese Maßnahme hat den Vorteil, dass das Gehäuse einen kompakten und stabilen Schutz für das Bildleitsystem bietet. Zudem erlaubt das Gelenkgehäuse dem Arzt während des Eingriffs eine gute Handhabbarkeit des Endoskops. Das Achsenkreuz zwischen beiden Gehäuseteilen ermöglicht die Abwinklung des Okulars bezüglich des Schafts in zumindest zwei zueinander quer angeordneten Ebenen.

In einer alternativen bevorzugten Ausgestaltung weist das Gelenk zumindest zwei voneinander beabstandete 1-Achsgelenke auf.

Diese Maßnahme stellt eine weitere technisch einfach realisierbare und kostengünstige Ausgestaltung des Gelenks dar, um eine räumliche Abwinkelung des Okulars relativ zur Längsrichtung des Schafts zu ermöglichen.

In einer weiteren bevorzugten Ausgestaltung weist das Gelenk einen Durchgang zur Durchführung des Bildleitsystems auf, wobei der Durchgang frei von Vorsprüngen ist.

Diese Maßnahme hat den Vorteil, dass das Bildleitsystem beim Abwinkeln des Okulars vor Beschädigungen durch die Innenseite des Gelenks geschützt wird.

In einer weiteren bevorzugten Ausgestaltung weist das Gelenk einen Schutzschlauch für das Bildleitsystem auf, wobei sich der Schutzschlauch durch den Durchgang des Gelenks erstreckt und das Bildleitsystem aufnimmt.

Diese Maßnahme hat den Vorteil, dass das empfindliche Bildleitsystem durch den Schutzschlauch noch besser vor Beschädigungen geschützt wird.

In einer weiteren bevorzugten Ausgestaltung erstreckt sich der Schutzschlauch vollumfänglich um das Bildleitsystem.

Diese Maßnahme hat den Vorteil, dass das Bildleitsystem nach allen Seiten gleichermaßen gleichmäßig vor Beschädigungen geschützt ist.

Weitere Vorteile und Merkmale ergeben sich aus der nachfolgenden Beschreibung und den beigefügten Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in den Zeichnungen dargestellt und werden in der nachfolgenden Beschreibung näher erläutert. Es zeigen:
- Fig. 1: eine perspektivische Ansicht eines Endoskops mit einem Endoskopkopf und mit einem nur teilweise dargestellten Schaft;
- Fig. 2A, 2B, 2C: jeweils eine Seitenansicht des Endoskops in Fig. 1, wobei Fig. 2A das Endoskop mit nicht abgewinkeltem Okular und Fig. 2B und 2C das Endoskop in verschieden abgewinkelten Stellungen des Okulars zeigt;
- Fig. 3: einen Querschnitt des Endoskopkopfes in Fig. 1 in nicht abgewinkelter Stellung;
- Fig. 4A, 4B: jeweils einen Querschnitt des Endoskopkopfes in Fig. 1 im Bereich des Gelenks in einer abgewinkelten (Fig. 4A) und einer nicht abgewinkelten Stellung (Fig. 4B);
- Fig. 5A, 5B: eine Seitenansicht eines Endoskopkopfes (Fig. 5A) gemäß eines weiteren Ausführungsbeispiels und eine perspektivische Explosionszeichnung des Gelenks (Fig. 5 B); und
- Fig. 6A, 6B: eine Seitenansicht des Endoskopkopfes (Fig. 6A) gemäß eines noch weiteren Ausführungsbeispiels und eine perspektivische Explosionszeichnung des Gelenks (Fig. 6B).

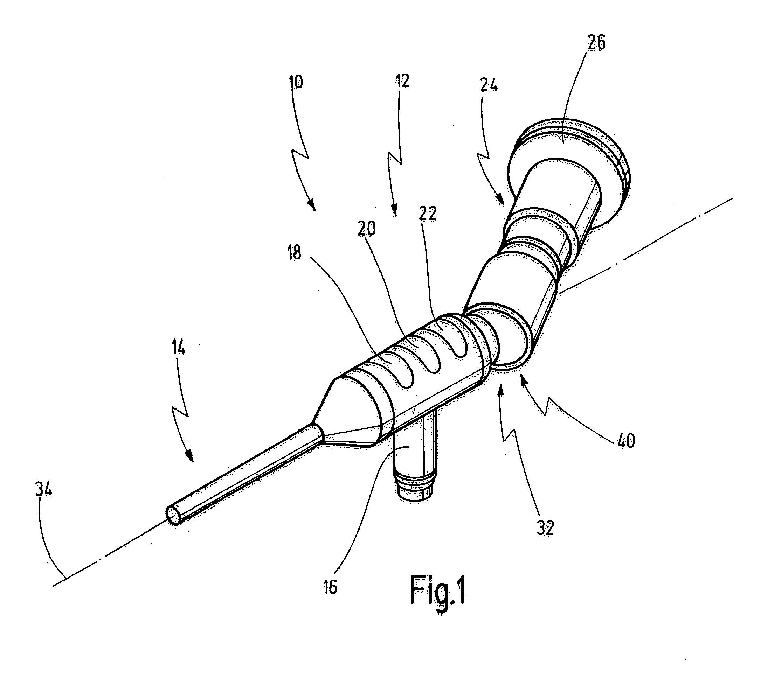

In Fig. 1 bis 3 ist ein mit dem allgemeinen Bezugszeichen 10 versehenes Endoskop zur Intubation eines Atemweges dargestellt.

Das Endoskop 10 weist an seinem proximalen Ende einen Endoskopkopf 12 und an seinem distalen Ende einen semi-flexiblen oder starren Endoskopschaft 14 auf. Das distale Ende des Endoskopkopfes 12 weist einen Anschluss 16 für ein Lichtleitkabel auf. Darüber hinaus ist das distale Ende des Endoskopkopfes 12 derart mit Einbuchtungen 18-22 ausgebildet, dass der Arzt seine Fingerspitzen in diese Einbuchtungen 18-22 legen kann, während er das Endoskop 10 hält. Das proximale Ende des Endoskopkopfes 12 weist einen Fokussierring 24 und eine Augenmuschel 26 auf, die am äußersten proximalen Ende des Endoskopkopfes 12 angeordnet sind.

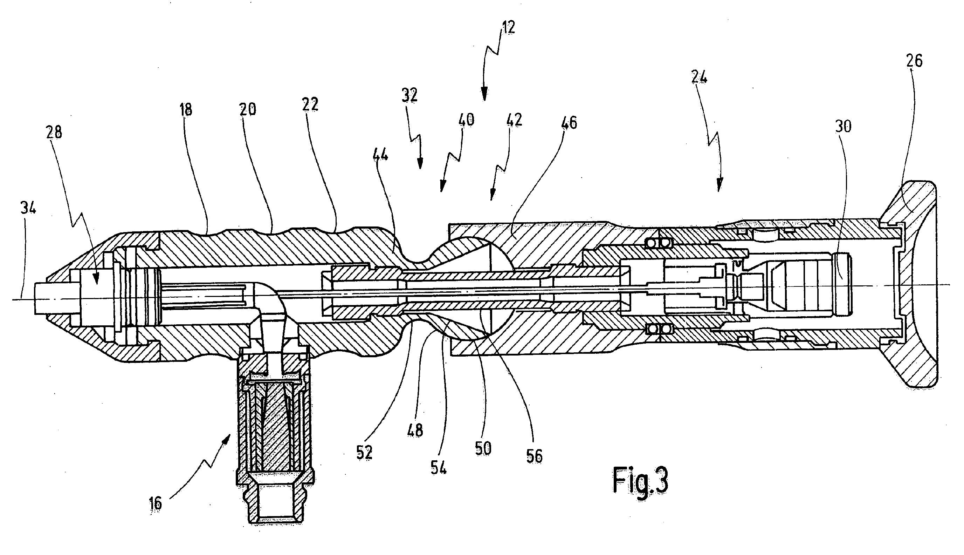

Wie in Fig. 3 dargestellt, verläuft ein Bildleitsystem 28 vom distalen Ende des Endoskopschafts 14 bis zum Okular 30, das am proximalen Ende des Endoskopkopfes 12 angeordnet ist.

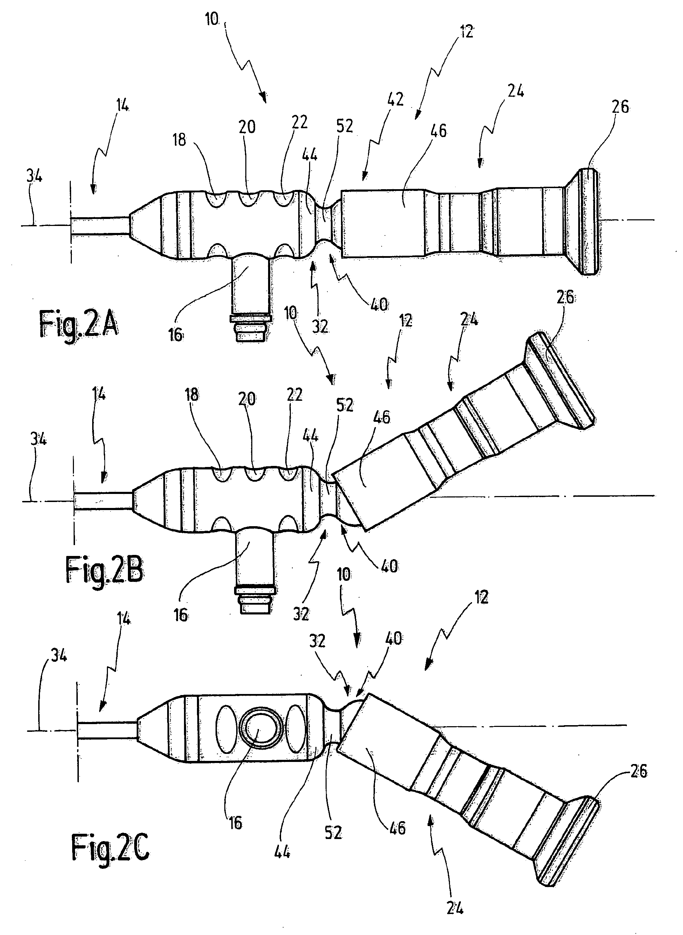

Zwischen dem Schaft 14 und dem Okular 30 ist ein Gelenk 32 angeordnet, so dass das Okular 30 relativ zur Längsrichtung 34 des Schafts 14 abwinkelbar ist. Die Längsrichtung 34 des Schafts 14 bezeichnet die Richtung, die durch das proximale Ende des Schafts 14 definiert wird. Das zumindest zweielementige Gelenk 32 ist derart ausgestaltet, dass das Okular 30 in einer ersten Ebene 36 relativ zur Längsrichtung 34 des Schafts 14 abwinkelbar ist. Darüber hinaus ist das Gelenk 32 in einer zweiten Ebene 38 quer zur ersten Ebene 36 abwinkelbar ausgestaltet. In Fig. 2B und 2C entsprechen diese Ebenen 36, 38 den jeweiligen Zeichenebenen. Der maximale Ablenkwinkel beträgt in einer bevorzugten Ausgestaltung jeweils etwa 50° zu beiden Seiten der Längsrichtung 34 des Schafts 14.

In einer ersten Ausgestaltung ist das Gelenk 32 als Kugelgelenk 40 mit einem zweiteiligen Gehäuse 42 ausgebildet (siehe Fig. 1 bis 4). Ein Gehäuseteil 44 ist mit dem Schaft 14, und ein zweites Gehäuseteil 46 ist mit dem Okular 30 verbunden. Das erste Gehäuseteil 44 weist eine kugelförmige Außenfläche 48 auf, die mit Reibschluss gleitend in einer kugelförmigen Innenfläche 50 des zweiten Gehäuseteils 46 aufgenommen ist. Der Reibschluss bietet den Vorteil, dass das zweite Gehäuseteil selbsthemmend im ersten Gehäuseteil 46 gehalten wird und eine abgewinkelte Stellung des Okulars 30 relativ zur Längsrichtung 34 des Schafts 14 beibehält, auch wenn der Arzt das zweite Gehäuseteil 46 nicht festhält. Die kugelförmige Innenfläche 50 des zweiten Gehäuseteils 46 erstreckt sich über den maximalen Außendurchmesser der kugelförmigen Außenfläche 48 des ersten Gehäuseteils 44. Diese Ausgestaltung hat den Vorteil, dass sich beide Gehäuseteile 44, 46 gegenseitig ohne weiteren technischen Aufwand halten.

Das erste Gehäuseteil 44 weist ferner eine radiale Einschnürung 52 an einer vom zweiten Gehäuseteil 46 abgewandten Seite auf. Die radiale Einschnürung 52 verjüngt sich in Längsrichtung 34 des Schafts 14 vom Kugelgelenk 40 weg gesehen. Die radiale Einschnürung 52 folgt der kugelförmigen Oberfläche des ersten Gehäuseteils 44. Die radiale Einschnürung 52 vergrößert den Ablenkwinkel des zweiten Gehäuseteils 46 relativ zur Längsrichtung 34 des Schafts 14, da das zweite Gehäuseteil 46 über einen größeren Winkelbereich nicht an dem ersten Gehäuseteil 44 anstößt. Das Kugelgelenk 40 erlaubt eine Ablenkung des Okulars 30 relativ zur Längsrichtung 34 des Schafts 14 in allen Ebenen, die durch den Mittelpunkt des kugelförmigen proximalen Endes des ersten Gehäuseteils 44 verlaufen. Der maximale Ablenkwinkel des Okulars 30 relativ zur Längsrichtung 34 des Schafts 14 ist dadurch gegeben, dass beide Gehäuseteile 44, 46 als Anschlag zusammen wirken, d.h. das zweite Gehäuseteil 46 stößt ab einem bestimmten Ablenkwinkel am ersten Gehäuseteil 44 an. Auf diese Weise wird verhindert, dass das Bildleitsystem 28 im Bereich des Gelenks 32 durchgebrochen wird.

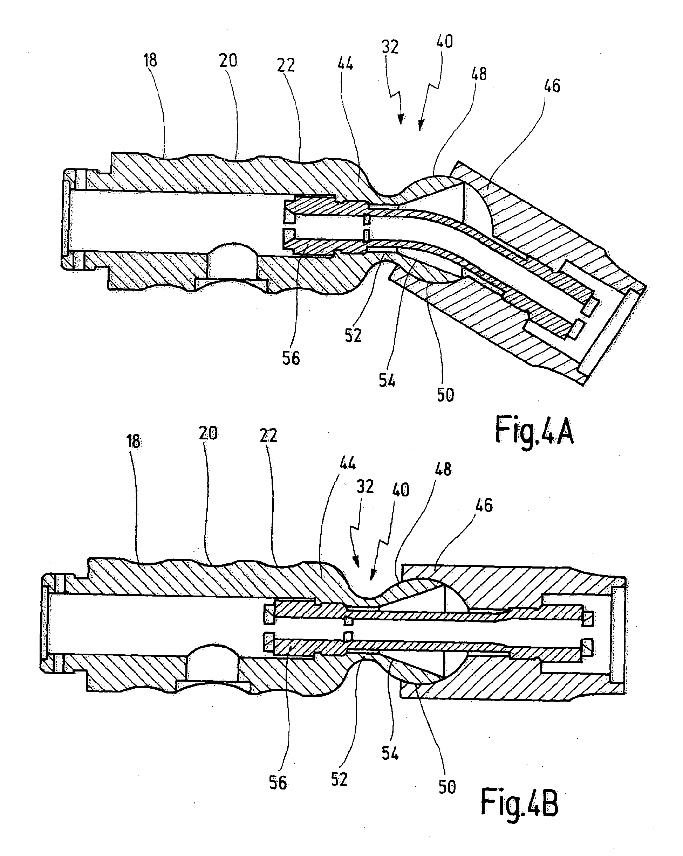

Wie in Fig. 3, 4 dargestellt, weist das Gelenk 32 ferner einen Durchgang 54 zur Durchführung eines Schutzschlauchs 56 auf, in dem das Bildleitsystem 28 aufgenommen ist. Der Übersicht halber ist das Bildleitsystem 28 in Fig. 4 nicht gezeigt. Der Durchgang 54 ist derart ausgestaltet, dass seine Oberfläche frei von Vorsprüngen ist. Hierdurch wird eine Beschädigung des Bildleitsystems 28 durch die Innenseite des Gelenks 32 verhindert. Darüber hinaus bietet der Schutzschlauch 56 zusätzlichen Schutz vor Beschädigungen des Bildleitsystems 28 durch das Gelenk 32. Der Schutzschlauch 56 ist derart ausgestaltet, dass er das Bildleitsystem 28 vollumfänglich aufnimmt und das Bildleitsystem 28 von allen Seiten gleichermaßen vor Beschädigungen durch das Gelenk 32 schützt.

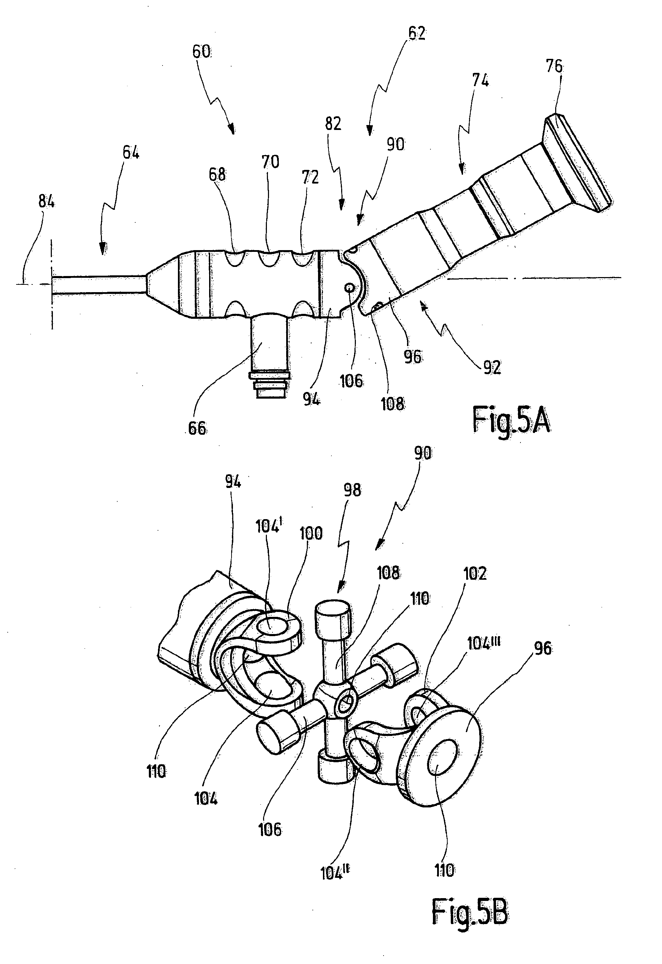

In Fig. 5A und 5B ist ein Endoskop 60 dargestellt, wobei die Merkmale des Endoskops 60, die mit denjenigen des Endoskops 10 gleich sind oder diesen entsprechen, mit um 50 erhöhten Bezugszeichen des Endoskops 10 versehen sind.

Ein Okular 80 ist in einer ersten Ebene 86 relativ zu einer Längsrichtung 84 eines Schafts 64 und in einer zweiten Ebene 88, die quer zur ersten Ebene 86 verläuft, abwinkelbar. Die Abwinkelung wird durch ein als Kardangelenk 90 ausgebildetes Gelenk 82 ermöglicht.

Das Kardangelenk 90 weist ein zweiteiliges Gehäuse 92 auf, wobei ein erstes Gehäuseteil 94 mit dem Schaft 64 und ein zweites Gehäuseteil 94 mit dem Okular 80 verbunden ist. Zwischen beiden Gehäuseteilen 94, 96 ist ein Achsenkreuz 98 angeordnet. Beide Gehäuseteile 94, 96 weisen je ein bogenförmiges Element 100, 102 auf, die quer zueinander angeordnet sind und ineinander greifen. Jedes bogenförmige Element 100, 102 weist je zwei sich gegenüberliegende Öffnungen 104-104' auf. Das Achsenkreuz 98 weist zwei zueinander quer verlaufende Befestigungsstifte 106, 108 auf, die in ihrem jeweiligen Mittelpunkt miteinander verbunden sind. Die Enden der Befestigungsstifte 106, 108 sind in den Öffnungen 104-104' der bogenförmigen Elemente 100, 102 aufgenommen. Das Kardangelenk 90 weist wie das Kugelgelenk 40 einen Durchgang 110 durch das Gelenk 82 auf. Ein Schutzschlauch 112 ist durch den Durchgang 110 des Kardangelenks 90 geführt. Der Übersicht halber ist der Schutzschlauch 112 nicht in Figur 5B dargestellt.

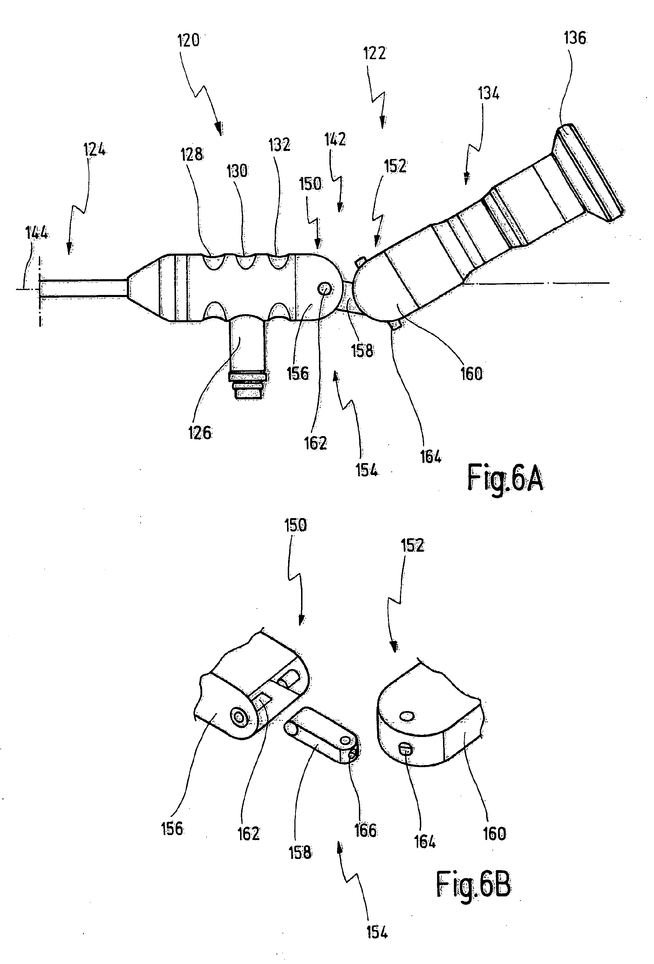

In Fig. 6A und 6B ist ein Endoskop 120 dargestellt, wobei die Merkmale des Endoskops 120, die mit denjenigen des Endoskops 10 gleich sind oder diesen entsprechen, mit um 110 erhöhten Bezugszeichen des Endoskops 10 versehen sind.

Ein Okular 140 ist in einer ersten Ebene 146 relativ zu einer Längsrichtung 144 eines Schafts 124 und in einer zweiten Ebene 148, die quer zur ersten Ebene 146 verläuft, abwinkelbar. Die Abwinkelung wird durch als zumindest zwei voneinander beabstandete 1-Achsgelenke 150, 152 ausgebildetes Gelenk 142 ermöglicht.

In der gezeigten Darstellung weist das Gelenk 142 ein dreiteiliges Gehäuse 154 auf, wobei ein erstes Gehäuseteil 156 mit dem Schaft 124 und über das 1-Achsgelenk 150 mit einem zweiten Gehäuseteil 158 verbunden ist. Das zweite Gehäuseteil 158 ist über das zweite 1-Achsgelenk 152 mit einem dritten Gehäuseteil 160 verbunden. Das dritte Gehäuseteil 160 ist mit einem Okular 140 verbunden. Jedes 1-Achsgelenk 150, 152 weist je einen Stift 162, 164 auf, der die sich anschließenden Gehäuseteile 156, 158, 160 gelenkig miteinander verbindet. Da beide Stifte 162, 164 quer zueinander angeordnet sind, ist das erste 1-Achsgelenk 150 in einer Ebene 146 relativ zu einer Längsrichtung 144 des Schafts 124 und in einer zweiten Ebene 146 quer zur ersten Ebene 144 abwinkelbar. In der gezeigten Darstellung der beiden 1-Achsgelenke 150, 152 sind die Stifte 162, 164 derartig durchbrochen, dass ein Durchgang 166 zur Aufnahme eines Lichtleitsystems 138 durch das Gelenk verläuft. Ein Schutzschlauch 168 ist durch den Durchgang 166 des Gelenks 142 geführt. Der Übersicht halber ist der Schutzschlauch 168 nicht in Figur 6B dargestellt.

## Patentansprüche

1. Endoskop, insbesondere zur Intubation eines Atemwegs, mit einem Schaft (14; 64; 124) und einem Okular (30; 80; 140) am proximalen Ende eines Endoskopkopfes (12; 62; 122) und mit einem Gelenk (32; 82; 142) zwischen dem Schaft (14; 64; 124) und dem Okular (30; 80; 140) zum Abwinkeln des Okulars (30; 80; 140) relativ zu einer Längsrichtung (34; 84; 144) des Schafts (14; 64; 124), wobei das Gelenk (32; 82; 142) so ausgebildet ist, dass das Okular (30; 80; 140) in einer ersten Ebene (36; 86; 146) abwinkelbar ist, **dadurch gekennzeichnet, dass** das Gelenk (32; 82; 142) so ausgebildet ist, dass das Okular (30; 80; 140) zumindest in einer zweiten Ebene (38; 88; 148), die quer zur ersten Ebene (36; 86; 146) verläuft, abwinkelbar ist.

2. Endoskop nach Anspruch 1, **dadurch gekennzeichnet, dass** das Gelenk (32; 82; 142) so ausgebildet ist, dass das Okular (30; 80; 140) zumindest in einer der ersten (36; 86; 146) oder zweiten Ebene (38; 88; 148) bis zu einem Winkel von etwa 50° relativ zur Längsrichtung (34; 84; 144) des Schafts (14; 64; 124) abwinkelbar ist.

3. Endoskop nach Anspruch 1 oder 2, **dadurch gekennzeichnet, dass** das Gelenk (32; 82; 142) so ausgebildet ist, dass das Okular (30; 80; 140) zumindest in einer der ersten (36; 86; 146) oder zweiten Ebene (38; 88; 148) bis zu einem Winkel von etwa 40° relativ zur Längsrichtung (34; 84; 144) des Schafts (14; 64; 124) abwinkelbar ist.

4. Endoskop nach einem der Ansprüche 1 bis 3, **dadurch gekennzeichnet, dass** das Gelenk (32; 82; 142) so ausgebildet ist, dass das Okular (30; 80; 140) zumindest in einer der ersten (36; 86; 146) oder zweiten Ebene (38; 88; 148) bis zu einem Winkel von etwa 30° relativ zur Längsrichtung (34; 84; 144) des Schafts (14; 64; 124) abwinkelbar ist.

5. Endoskop nach einem der Ansprüche 1 bis 4, **dadurch gekennzeichnet, dass** das Gelenk (32) als Kugelgelenk (40) ausgebildet ist.

6. Endoskop nach Anspruch 5, **dadurch gekennzeichnet, dass** das Kugelgelenk (40) ein zweiteiliges Gehäuse (42) aufweist, wobei ein erstes Gehäuseteil (44) mit dem Schaft (14) und ein zweites Gehäuseteil (46) mit dem Okular (30) verbunden ist.

7. Endoskop nach Anspruch 6, **dadurch gekennzeichnet, dass** das erste Gehäuseteil (44) mit Reibschluss gleitend in dem zweiten Gehäuseteil (46) aufgenommen ist.

8. Endoskop nach Anspruch 6 oder 7, **dadurch gekennzeichnet, dass** das erste Gehäuseteil (44) eine kugelförmige Außenfläche (48) und das zweite Gehäuseteil (46) eine kugelförmige Innenfläche (50) aufweist, in der das erste Gehäuseteil (44) aufgenommen ist.

9. Endoskop nach einem der Ansprüche 6 bis 8, **dadurch gekennzeichnet, dass** das erste Gehäuseteil (44) derart in dem zweiten Gehäuseteil (46) angeordnet ist, dass sich das zweite Gehäuseteil (46) über den maximalen Außendurchmesser der kugelförmigen Außenfläche (48) des ersten Gehäuseteils (44) hinweg erstreckt.

10. Endoskop nach Anspruch 8 oder 9, **dadurch gekennzeichnet**, das die kugelförmige Außenfläche (48) des ersten Gehäuseteils (44) eine radiale Einschnürung (52) auf einer dem zweiten Gehäuseteil (46) abgewandten Seite aufweist.

11. Endoskop nach Anspruch 10, **dadurch gekennzeichnet, dass** sich die radiale Einschnürung (52) in Längsrichtung vom Gelenk (32) weg gesehen verjüngt.

12. Endoskop nach einem der Ansprüche 6 bis 11, **dadurch gekennzeichnet, dass** beide Gehäuseteile (44, 46) als Anschlag zur Begrenzung der Abwinkelung des Okulars (30) zusammen wirken.

13. Endoskop nach einem der Ansprüche 1 bis 4, **dadurch gekennzeichnet, dass** das Gelenk (82) als Kardangelenk (90) ausgebildet ist.

14. Endoskop nach Anspruch 13, **dadurch gekennzeichnet, dass** das Kardangelenk (90) ein zweiteiliges Gehäuse (92) und ein Achsenkreuz (98) aufweist, wobei ein erstes Gehäuseteil (94) mit dem Schaft (64) und ein zweites Gehäuseteil (96) mit dem Okular (82) verbunden ist und das Achsenkreuz (98) zwischen den beiden Gehäuseteilen (94, 96) angeordnet ist.

15. Endoskop nach einem der Ansprüche 1 bis 4, **dadurch gekennzeichnet, dass** das Gelenk (142) zumindest zwei voneinander beabstandete 1-Achsgelenke (150, 152) aufweist.

16. Endoskop nach einem der Ansprüche 1 bis 15, **dadurch gekennzeichnet, dass** das Gelenk (32; 82; 142) einen Durchgang (54; 110; 166) zur Durchführung eines Bildleitsystems (28; 78; 138) aufweist, wobei der Durchgang (54; 110; 166) frei von Vorsprüngen ist.

17. Endoskop nach Anspruch 16, **dadurch gekennzeichnet, dass** das Gelenk (32; 82; 142) einen Schutzschlauch (56; 112; 168) für das Bildleitsystem (28; 78; 138) aufweist, wobei sich der Schutzschlauch (56; 112; 168) durch den Durchgang (54; 110; 166) des Gelenks (32; 82; 142) erstreckt und das Bildleitsystem (28; 78; 138) aufnimmt.

18. Endoskop nach Anspruch 17, **dadurch gekennzeichnet, dass** sich der Schutzschlauch (56; 112; 168) vollumfänglich um das Bildleitsystem (28; 78; 138) erstreckt.
